**Europäisches Patentamt**

**European Patent Office**

**Office européen des brevets**

(19)

(11) Publication number: **0 271 214**
**A2**

(12)

# EUROPEAN PATENT APPLICATION

(21) Application number: **87309913.9**

(22) Date of filing: **10.11.87**

(51) Int. Cl.⁴: **A61B 5/20 , A61B 8/08**

The title of the invention has been amended (Guidelines for Examination in the EPO, A-III, 7.3).

(30) Priority: **13.11.86 US 929869**

(43) Date of publication of application:
**15.06.88 Bulletin 88/24**

(84) Designated Contracting States:
**BE CH DE FR GB IT LI SE**

(71) Applicant: **NATIONAL AERONAUTICS AND SPACE ADMINISTRATION**
**NASA Headquarters**
**Washington, D.C. 20546(US)**

(72) Inventor: **Companion, John Alexander**
**20A Curtis Lane**
**Hampton Virginia 23669(US)**
Inventor: **Heyman, Joseph Saul**
**130 Indian Springs Road**
**Williamsburg Virginia 23185(US)**
Inventor: **Blalock, Travis Nelson**
**1009 Laurel Hill Road**
**Knoxville Tennessee 37923(US)**
Inventor: **Mineo, Beth Ann**
**1800 Old Pond Drive**
**Arlington Texas 76011(US)**
Inventor: **Cavalier, Albert Ralph**
**1838 Carriage House Circle**
**Arlington Texas 76011(US)**

(74) Representative: **Barnard, Eric Edward et al**
**BROOKES & MARTIN High Holborn House**
**52/54 High Holborn**
**London WC1V 6SE(GB)**

(54) Apparatus for measuring the volume of urine in the human bladder.

(57) A device and method for the rapid quantification of an internal property of a material, typically the amount of urine in a human bladder. Ultrasonic transducer 1 is positioned in proximity to the bladder 12-13, and excited by pulser/receiver 3 under command of logic 4 to launch an acoustic wave into patient 2. This wave interacts with the bladder walls 12, 13 and is reflected back to the transducer whence it is amplified and processed by pulser/receiver 3. The resulting signal is digitized by analog-to-digital converter 5 under command of logic 4, and stored in memory 6. The software in logic system 4 determines the amount of urine in the bladder as a function of the propagated ultrasonic energy and based on programmed scientific measurements and past history with the specific subject, and actuates an alarm.

FIGURE 1

## RAPID QUANTIFICATION OF AN INTERNAL PROPERTY

### Background of the Invention

This invention relates generally to the rapid quantification of an internal property of a material as a function of energy transmitted into the material and reflected therefrom, and particularly to a method and a device for rapidly quantifying the amount of urine in the bladder of a human subject as a function of ultrasonic energy transmitted into the subject and reflected therefrom.

There has been a long-standing need for a method and a device for the rapid quantification of internal properties of materials, such as the amount of urine in the bladders of human subjects, especially the mentally retarded, the infirm, and the elderly.

In attempts to normalize the lives of persons with mental retardation, much energy has been devoted to teaching these persons to function independently in society. The problem of incontinence often thwarts the best of these efforts. While sophisticated toilet training programs are successful in teaching some persons what to do once the internal sensation of a full bladder is perceived, these programs typically presuppose that a person is capable of realizing when she/he has to void. The subset of the population of persons with mental retardation for whom continued incontinence is a more common problem are those persons with severe or profound mental retardation, i.e., those with IQs less than 35 and significant deficits in adaptive behavior, who have difficulty in recognizing the subtle and somewhat obscure signals of their bladders.

In addition, there is a substantial need to provide increased independence for persons who have permanently lost the ability to control their bladders for medical reasons such as diabetes, cerebral palsy, quadriplegia, spina bifida, and advanced age.

Incontinence typically results in a negative stigma for the person, reduced positive interaction with other people, unsanitary living conditions, excessive laundry expenses, and increased custodial attention by caregivers. Because of the failure to acquire fundamental toileting skills, such persons are often excluded from a wide variety of vocational, social, and recreational programs, in addition to many preschool programs--all of which are important components of overall experience necessary for their developmental growth and eventual integration into community life.

Previous attempts to employ technology in urinary toilet training fall into two classes. The first class is the wetness detector, which alerts the subject when urine is present on the person. A particular example of this is the employment of a moisture-sensitive apparatus in the clothing or in the bed, which device triggers an alarm when moisture is detected. The second class is the motion-sensitive device, which is located in the toilet. Once voiding has begun, the motion imparted to this device helps the user recognize that urination has been initiated. However, both classes of devices produce their effects after urination has taken place. That is to say, their users are helped to recognize when voiding has been initiated, but they are not helped to recognize the preliminary need to urinate, and thereby make the association between this need and socially-acceptable toileting behavior. In neither case is there a quantification of the amount of urine in the bladder, which would be of significance in helping one to recognize the preliminary need to urinate.

Urologists have recently employed an ultrasonic device which scans the entire bladder and images it with a sector scan to show the extent of the bladder wall over a sixty degree angle. Other recently-developed devices are based on an ultrasonic "A" scan to reveal the position of the back wall of the bladder, which moves during inflation. However, the scanning systems are costly, not portable, and require extensive training to use and interpret. Moreover, the "A" scan devices can only reveal the position of the back wall of the bladder. Since the bladder is not a sphere, rectangle, or other simple geometric shape, its volume increases as a complex function, which is dependent on much more information than merely one of its dimensions (viz., that of the back wall). Accordingly, such devices can only provide a relative urine volume while the bladder is partially inflated, and they are useless when the bladder is nearing its maximum size, and the normal urge to eliminate is strongest. In short, these devices do not quantify the amount of urine in the bladder and therefore do not provide information needed by the subject during the critical time when the bladder is at or near its full extension.

### Summary of the Invention

The invention comprehends the provision of a transducer, which is positioned in proximity to the material under test, for the purpose of transmitting energy into the material followed by receiving energy reflected from the material. An ultrasonic transducer is especially useful, particularly when the material under test is a container which is being filled or emptied, such as an organ of a living being, especially the bladder of a human subject. A pulser/receiver communicates with a source of power and the transducer and excites the transducer to transmit energy. It also amplifies and processes the reflected energy received by the transducer. A converter communicates with the pulser/receiver to digitize the analog signal from the pulser/receiver. A memory communicates with the converter for storage of the digital signal from the converter. A logic system communicates with the pulser/receiver to command excitation of the transducer. The logic system communicates additionally with the converter to command digitization of the analog signal from the pulser/receiver. The logic system also communicates with the memory and processes the stored digital signal from the converter along with digital input based on the particular characteristics of the specific material under test, thereby rapidly quantifying the internal property of the material as a function of the propagated energy. When the material under test is a container being filled or emptied, such as an organ of a living being, especially the bladder of a human subject, it is advantageous to provide an alarm to alert the subject or another person at a remote location when the amount of urine in the bladder has reached a given level.

Brief Description of the Drawings

Figure 1 is a schematic showing the components of a preferred embodiment of the invention;

Figures 2-4 are schematics illustrating the interaction of acoustic waves with the bladder of a human subject in accord with the preferred embodiment; and

Figure 5 is a schematic and Figure 6 is a graph illustrating in detail the function and operation of the components of the preferred embodiment of Figure 1.

Detailed Description of the Invention

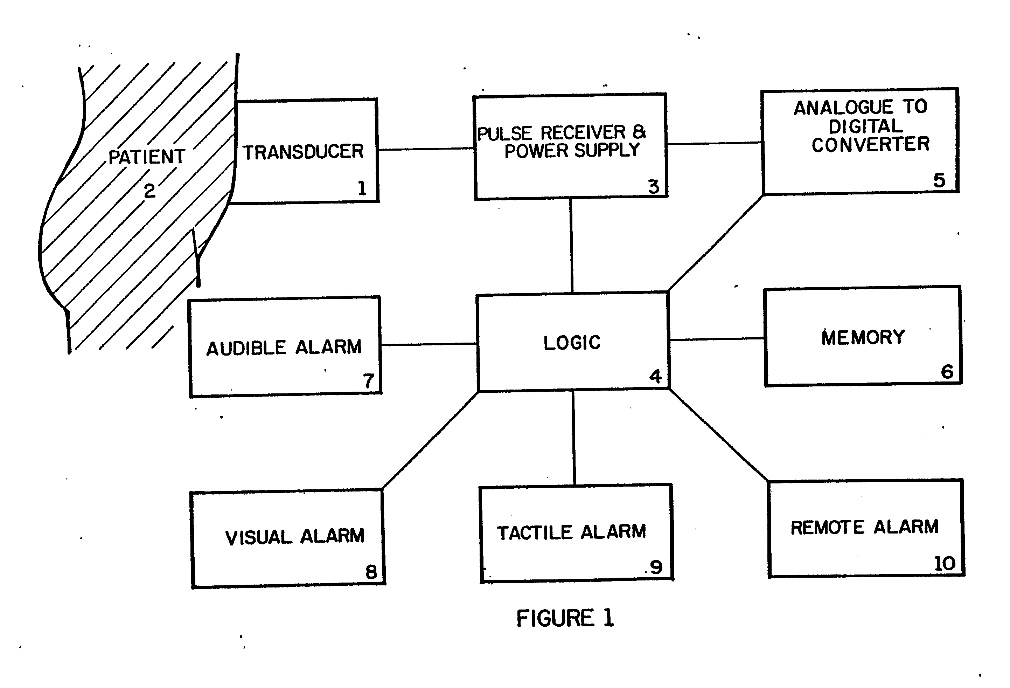

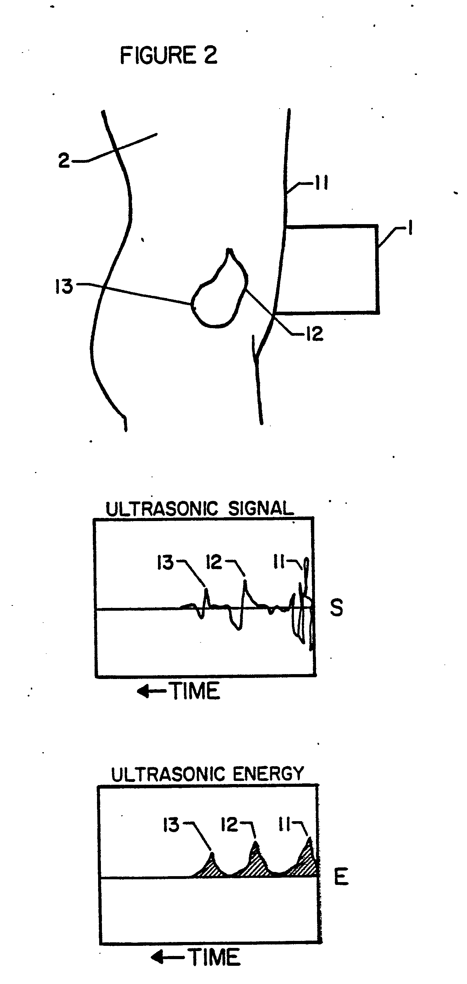

Figure 1 shows an ultrasonic transducer 1, which is excited by pulser/receiver 3 under command of logic 4 to launch an acoustic wave into patient 2. The wave interacts with the bladder wall (see Figures 2-4 and discussion infra.) and is reflected back to the ultrasonic transducer 1. It is then amplified and processed by pulser/receiver 3. The resulting signal is digitized by the analog-to-digital converter 5 under command of logic 4, and is stored in memory 6. The software in the logic system 4 determines the bladder fullness based on scientific measurements and past history with the specific patient, and sends out a signal to turn on any or all of the audible 7, the visible 8, the tactile 9, and the remote wireless alarm 10.

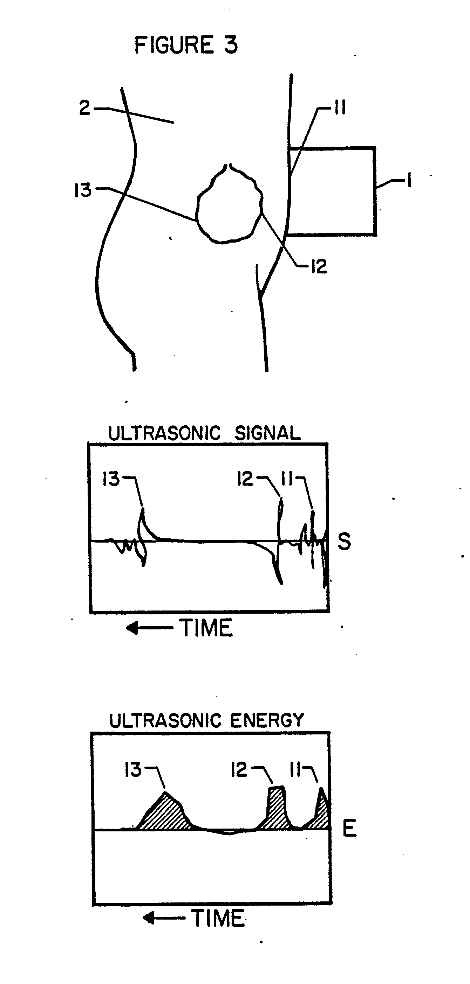

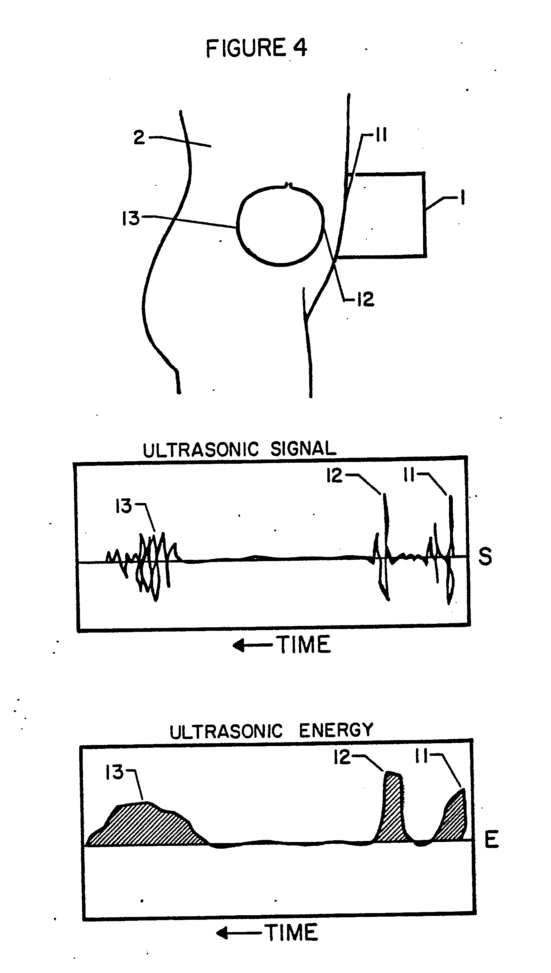

To better understand the function and operation of the invention, it is necessary to examine the acoustic wave interaction with the bladder as is shown in Figures 2-4. In Figure 2, the bladder is essentially empty. In Figure 3, the bladder is being filled, and in Figure 4, the bladder is at maximum fullness. In each of Figures 2-4 are simplified, illustrative diagrams of the physical bladder and the ultrasonic transducer (top), a conventional ultrasonic signal S showing the radio frequency (RF) waveforms (middle), and the energy waveforms E (bottom). Each of Figures 2-4 shows the tissue/transducer interface 11, the bladder front wall 12, and the bladder back wall 13.

In Figure 2, with the bladder essentially empty, transducer 1 is placed on the patient with a conventional couplant for ultrasound. The sound wave excited by the pulser/receiver 3 of Figure 1 causes the ultrasonic signal shown in Figure 2, diagram S, time position 11. The wave also reflects off the bladder front wall 12 and the bladder back wall 13, with the resulting ultrasonic signals 12 and 13, respectively, in diagram S. The bottom diagram E in Figure 2 is the ultrasonic energy with its corresponding signals 11, 12, and 13. These signals are obtained by adding the absolute amplitude of the RF waveforms for each pulse and averaging the resulting summation over N cycles of the measurement, as determined by the programmed logic. It should be noted that the energy is positive definite.

In Figure 3, with the bladder partially full, the bladder has inflated as shown in the upper part of the figure, and the RF waveforms have changed as the bladder shape has changed. In particular, the back wall reflection has moved back in time, and additional reverberation has built up in the back wall signal.

In Figure 4, with the bladder full, the change in shape has continued, although the back wall has not moved in a simple fashion during filling. The energy seen in the back wall reflection, however, continued to increase as the bladder filled. In fact, for a bladder filling past about 60% fullness, the back wall hardly moves at all, while the energy reverberation at the back wall continues to increase. Thus, it can be seen that a monitor of the back wall position only would not be accurate during critical near-full periods. In sharp contrast thereto, this invention, which measures the energy in the back wall reflection as well as the back wall position, is accurate as a monitor for the entire range of bladder fullness.

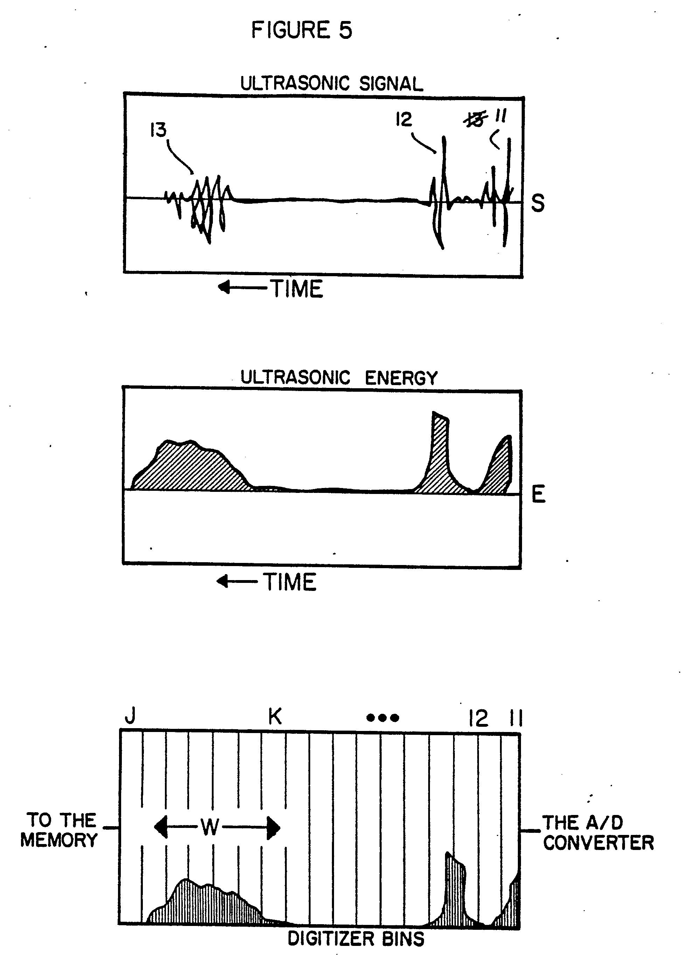

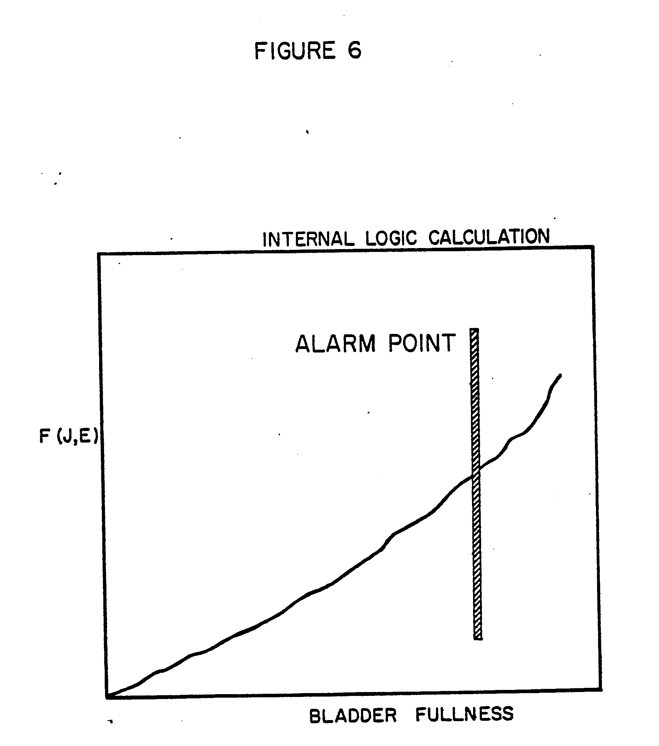

Figures 5 and 6 show the internal components of this invention and their function in more detail. The converter 5 and the memory 6 actually act as a signal averager, taking the digitizer output and multiplying it by some function of bin number and energy F(J,E), while checking that the signal falls in the correct time range or bin number J. The entire operation is controlled by the software to configure the function and the mathematical operations for the specific subject.

As the simplest case, the function used is the sum of energy amplitudes in bins J-K+W that correspond to the back wall and beyond of the bladder, where W is the width of the reverberation signal at the back wall. A check on the data quality is that bins less than J-K show no significant amplitude. Such a lack of signal corresponds to the fact that when the bladder contains water, the path length between the front and back walls should shown no scattering, i.e., a simple water path exists.

The internal logic calculation of Figure 6 shows the result of a typical bladder during filling. The function has been adapted to the specific subject so that the F(J,E) and the alarm point correspond to the best time for that subject to be notified to urinate.

A complete electronics package is worn by the subject with the transducer positioned by means of a flexible mounting belt. The electronics package advantageously contains means to alert the subject with any of a variety of stimuli including a tactile alarm (e.g., a vibrator), a visual alarm (e.g., an LED mounted on eyeglasses), an audible alarm (e.g., a buzzer), and a remote alarm (an RF link to a receiver monitor). In addition, the electronics package advantageously contains a working mode which lets the package work in a "sleep" configuration when the bladder should be empty (after successful elimination). In that mode, the frequency of pulses and measurements is reduced to lengthen the life of the battery in the package. Moreover, parameters governing the user's interaction with the device, by the user or his/her caregiver into the logic system externally by adjusting controls on the face of the logic unit. This affords a customization for each individual and a quick and simple modification of existing parameters at any time. The user is accordingly allowed to select the level of bladder fullness at which she/he would like the alarm to sound.

**Claims**

1. A device for rapidly quantifying a relative distention of the bladder of a living subject, comprising:

an arrangement for transmitting an acoustical wave into the bladder of a living subject so as to create a reflected acoustical waveform;

an arrangement for measuring a time range together with an energy level of said reflected acoustical waveform; and

an arrangement for comparing said measurement against a standard, said standard being indicative of an amount of urine in the bladder of the living subject.

2. The device of claim 1, wherein said standard includes measurements conducted upon the living subject.

3. The device of claim 1, wherein said measuring arrangement includes an arrangement for producing an analog electrical signal of the time range and energy level of said reflected acoustical waveform and an arrangement for digitizing said analog electrical signal, said comparing arrangement including an arrangement for comparing the digitized analog signal against a digitized standard.

4. The device of claim 1, wherein said transmitting and measuring arrangements include;

an ultrasonic transducer adapted to be positioned on the external abdomen of a living subject in proximity to the bladder, said ultrasonic transducer adapted to emit acoustic waves into the subject and to receive reflected acoustic waves from the subject;

a pulser/receiver arrangement in communication with a source of power and the ultrasonic transducer for exciting the ultrasonic transducer to emit the acoustic waves and for amplifying and processing the reflected waves received by the ultrasonic transducer, and for providing analog signals representative of at least one reflected waveform over a respective time interval;

a converter in communication with the pulser/receiver arrangement for digitizing the analog signals from the pulser/receiver arrangement to provide corresponding digital signals representative of said at least one waveform;

said comparing arrangement including:

a memory arrangement in communication with the converter means for storing the corresponding digital signals therefrom, said memory arrangement also for storing digital input signals representative of a characteristic of the living subject, said characteristic related to the amount of urine in the bladder; and

a logic arrangement with the pulser/receiver arrangement for commanding excitation of the ultrasonic transducer; the logic arrangement also in communication with the converter for commanding the digitization of the analog signals from the pulser/receiver; the logic arrangement also in communication with the memory arrangement for receiving said digital input signals and said stored corresponding digital signals, said logic arrangement processing said corresponding digital signals to provide a function signal related to a value of the corresponding digital signals and their time of occurrence within said respective time intervals, and for comparing said function signal with said digital input signals, thereby quantifying the amount of urine in the bladder of the human subject as a function of propagated ultrasonic energy.

5. The device of claim 4, further comprising an alarm in communication with the logic system to indicate when the amount of urine in the bladder has reached a predetermined level.

6. The device of claim 5, wherein the alarm is adapted to be placed on or in proximity to the subject and includes at least one of an audible alarm, a visual alarm, and a tactile alarm.

7. The device of claim 5, wherein the alarm is adapted to be employed remotely from the subject and includes at least one of an audible alarm, a visual alarm, and a tactile alarm.

8. A method for rapidly quantifying a relative distention of the bladder of a living subject, comprising the steps of:

transmitting an acoustical wave into the bladder of a living subject so as to create a reflected acoustical waveform;

measuring a time range together with an energy level of said reflected acoustical waveform; and

comparing said measurement against a standard, said standard being indicative of an amount of urine in the bladder of the living subject.

9. The method of claim 8, wherein said step of obtaining a digital input signal includes using measurements conducted upon the living subject.

10. The method of claim 8, wherein said measuring step includes producing an analog electrical signal of said reflected acoustical waveform and digitizing said analog electrical signal, said comparing step including the step of comparing the digitized analog signal against a digitized standard.

11. The method of claim 8, wherein said transmitting and measuring steps include:

positioning an ultrasonic transducer externally on an abdomen of a living subject in proximity to the bladder;

emitting an acoustic wave into the living subject from the positioned ultrasonic transducer;

producing analog signals representative of at least one reflected waveform over a respective time interval;

digitizing the analog signals so as to produce first digital signals of said at least one reflected waveform;

obtaining digital input signals representative of a characteristic of the human subject, said characteristic related to an amount of urine in the bladder;

processing said first digital signals so as to obtain a function signal related to a value of said first digital signals and to their times of occurrence within said respective time interval; and

comparing said function signal with said digital input signals so as to quantify the amount of urine in the bladder of the living subject.

12. The method of claim 9 further comprising the step of electronically storing said first digital signals and said digital input signals.

FIGURE 1

0 271 214

# FIGURE 2

2

11

1

13

12

## ULTRASONIC SIGNAL

13   12      11

S

←TIME

## ULTRASONIC ENERGY

13   12   11

E

←TIME

## FIGURE 3

ULTRASONIC SIGNAL

←—TIME

ULTRASONIC ENERGY

←—TIME

## FIGURE 4

ULTRASONIC SIGNAL

←—TIME

ULTRASONIC ENERGY

←—TIME

# FIGURE 5

ULTRASONIC SIGNAL

13    12    13 11

S

←—TIME

ULTRASONIC ENERGY

E

←—TIME

J     K    •••   12   11

TO THE
MEMORY ←—    ←—W—→    —→ THE A/D
CONVERTER

DIGITIZER BINS

## FIGURE 6

INTERNAL LOGIC CALCULATION

ALARM POINT

F (J,E)

BLADDER FULLNESS